# EUROPEAN PATENT SPECIFICATION

(11) **EP 1 880 840 B1**
(45) Date of publication and mention of the grant of the patent: **30.06.2010**
(21) Application number: 07000395.9
(22) Date of filing: 03.09.2004
(51) Int. Cl.: B32B 5/18, A61F 13/15, D04H 13/00

(54) **Absorbent article**
Saugfähiger Artikel
Article absorbant

(30) Priority: 05.09.2003 US 656428
(43) Date of publication of application: 23.01.2008
(62) Divisional of application: 04255367.7
(73) Proprietor: Johnson and Johnson Consumer Companies, Inc., Skillman, NJ 08558-9418 (US)
(72) Inventor: Poccia, John F. III, Monmouth Beach NJ 07750 (US); Beauford, Shane D., Philadelphia PA 19149 (US)
(74) Representative: Mercer, Christopher Paul

(56) References cited:
- EP-A1- 0 403 187
- WO-A1-93/09741
- US-A- 4 341 217
- US-A1- 2002 115 953

## Description

### Field Of The Invention

The present invention relates to absorbent articles. The absorbent articles comprise an absorbent material which is laminated to an apertured film. Absorbent articles of the invention are useful as components of various absorbent products including, but not limited to, sanitary protection products, disposable diapers, and adhesive bandages.

### Background Of The Invention

Absorbent products such as sanitary napkins, disposable diapers and bandages are used to absorb body fluids such as menses, urine and wound exudate. These absorbent products generally require that the absorbent pad or absorbent article thereof be able to absorb a significant amount of body fluid. In some instances, the absorbent component must be capable of absorbing an amount of body fluid whose weight is greater than the weight of the absorbent material itself. It is also desirable that the body contacting surface of an absorbent product be dry, or relatively dry, after the absorbent product has absorbed the body fluid which it is designed to receive. It is further desirable that the absorbent component not stick to the skin or a wound site during use.

United States Patent Number 3,929,135 discloses an absorptive structure comprising an absorbent material and an apertured film. The absorbent material may be, for example, comminuted wood pulp. The apertured film serves as a top sheet for the absorptive structure. The apertured film is smooth on one side, and has protuberances on the other side. The protuberances of the apertured film face the absorbent material thereby forming the absorptive structure.

United States Patent Number 4,341,217 discloses a disposable absorbent article. The absorbent article has an absorbent core made of a material such as comminuted wood pulp- An apertured film having protuberances encloses the absorbent core. The protuberances of the apertured film face both major surfaces of the absorbent core.

United States Patent Number 6,600,085 discloses a disposable absorbent article. The absorbent article comprises an absorbent core sandwiched between two layers of apertured film having protuberances. On one side of the article, protuberances face the absorbent core. On the other side of the article, the protuberances face away from the absorbent core.

Despite the disclosure of the above-mentioned patents, there is a continuing need for an absorbent article with the ability to absorb a significant amount of fluid yet not slick to the skin or wounds during use.

### Summary Of The Invention

The present invention provides an absorbent article comprising an absorbent nonwoven fabric having a density from about 0.01 g/cc to about 0.05 g/cc, said absorbent nonwoven fabric having a first major surface and a second major surface; and an apertured film secured to at least one of the major surfaces of the absorbent nonwoven fabric. The apertured film can be secured to the said at least one major surface of the nonwoven fabric by frictional engagement, by an adhesive or by heat sealing, ultrasonic bonding or the like.

### Detailed Description Of The Preferred Embodiments

The absorbent article of the present invention comprises an apertured film. When the absorbent article is used as a non-stick wound-contacting pad for an adhesive bandage comprising a backing material and the absorbent article, the absorbent article is secured to the backing material so that when the adhesive bandage is used, the apertured film of the absorbent article contacts the wound. The apertured film has an open area. The apertured film optionally comprises protuberances. When the apertured film contains protuberances, the film is oriented such that the protuberances face the absorbent nonwoven fabric.

The apertured film may be an essentially flat film that contains apertures and has two relatively smooth major surfaces. This type of apertured film is commercially available, for example, as RETICULON^{™} brand film from PGI Inc.

Preferably, the apertured film used in the practice of the present invention is a macroscopically expanded film having a first major surface and a second major surface. The first major surface is relatively smooth. The second major surface comprises a plurality of protuberances which are formed in the film manufacturing process and extend outwardly from said second major surface. Apertured films of this type are disclosed in United States Patent No. 3,929,135. Apertured films of the type disclosed in United States Patent No. 3,929,135 are commercially available from Tredegar Inc. under the designations Tredegar X-6799, Tredegar X-6845, Tredegar X-6923, Tredegar X-6944 and Tredegar X-6844. Tredegar X-6944 and Tredegar X-6845 are particularly preferred.

Other apertured films, all useful in the practice of the present invention, are disclosed in United States Patent Number 4,324,246, United States Patent Number 4,342,314, United States Patent Number 4,463,045, and United States Patent Number 5,006,394.

If the absorbent article of the invention comprises an apertured film on each side of the absorbent nonwoven fabric, the same apertured film may be used on both sides-Alternatively, one type of apertured film can be used on one of the sides and a different type of apertured film can be used on the other side.

The apertured films may be made from any suitable polymeric material including, but not limited to, polyethylene, metallocene catalyzed polyethylene, polypropylene, and copolymers thereof. The apertured films may also be made from ethylene vinyl acetate copolymers and olefin alkylene copolymers, especially ethylene methyl acrylate copolymers.

When the laminated material of the present invention is used as a component of an adhesive bandage, an apertured film of one type may be used for the wound contacting surface of the adhesive bandage of the invention, while an apertured film of another type may be used for a backing material. Usually, however, the same apertured film is used for both the wound contacting surface and the backing material.

The apertured film may be three dimensional as disclosed in United States Patent No. 6,566,577. The apertured film typically is made from a thermoplastic material. The apertured film may be made from substantially any thermoplastic film-forming polymer. Preferably, the film is conformable but not substantially elastomeric- Preferably, the film is made from a hydrophillic polymer or the polymer is treated to be hydrophilic. Suitable polymers include, but are not limited to, polyethylene, polypropylene, polyester, polyamides such as nylons, fluoropolymers such as polyvinylidene fluoride or polytetrafluoroethylene, and mixtures thereof. Preferably, the film forming thermoplastic polymer is a copolymer of ethylene methyl acrylate.

Preferably, the thermoplastic film is textured and perforated. The term "textured" indicates that the film is patterned in relief, for example, patterned with protruding ridges or nubbles, for example by embossing as disclosed in United States Patent No. 6,566,577. The texturing renders the film less adherent to a wound. The ridges or nubbles are preferably rounded, and preferably project by 0.1 to 1.5 mm above the median plane of the film surface. More preferably they project by 0.2 to 1.0 mm above the median plane of the film.

Preferably, the thermoplastic film is both textured and perforated by means of mesh perforation. In this method, the film is supported on a reticulated mesh surface and heated to its softening temperature. Suction is then applied through the mesh. or air is blown onto the film above the mesh, which results in impression of the mesh into the film and the formation of perforations in the film at the interstices of the mesh. Mesh perforation techniques arc described in more detail in U.S. Pat. No. 3,054,148.

Preferably, the thermoplastic film has from 5 to 50 perforations/cm², more preferably from 10 to 30 perforations/cm². Preferably, the perforations have an area of from 0.01 to 2.0 mm². The hole-to-land ratio, defined as the ratio of the total area of the perforations to the total area of the film minus the area of the perforations, both areas being viewed in plans projection, is preferably from 0.01 to 1.0, more preferably from 0.05 to 0.5 and most preferably from 0.1 to 0.3.

The thermoplastic film should be as thin as possible consistent with the need for physical integrity during manufacture and use. Typically, the film has a basis weight of from 1 to 500 g/m², preferably from 10 to 200 g/m².

The open area of the apertured films utilized in the absorbent article of the invention is defined as the area occupied by apertures. The open area may range from about 1 percent to about 35 percent, preferably from about 4 percent to about 30 percent, of the total area of the apertured film.

The apertured film of the article of the invention may be treated with a hydrophilic surfactant including, but not limited to, laurate esters of sorbitol and sorbitol anhydrides condensed with ethylene oxide, such as polysorbate 20, polysorbate 40, polysorbate 60, and polysorbate 80; ethylene oxide/propylene oxide copolymers; octyl phenol ethoxylates; nonyl phenol ethoxylates; and ethoxylaled alcohols. The term "treated" means that the apertured film has had a hydrophillic surfactant incorporated therein during the polymerization process used to manufacture the polymeric resin from which the apertured film is made, or the hydrophilic surfactant is incorporated with the polymer during the process by which the apertured film is made, or the apertured film is coated with the hydrophillic surfactant after the apertured film has been made.

The apertured film may, if desired, comprise Triclosan or a like anti-bacterial agent in an anti-bacterially effective amount.

The absorbent article of the invention may further include a top layer. The top layer may comprise any polymeric film known in the art. Such polymeric films include, but are not limited to, polyolefin films, polyvinyl chloride films, the apertured films described above, a microporous film, and the like. Microporous films are preferred.

Microporous films are commercially available and arc described in United States Patent No. 6,595,042. Microporous films may be made from any polymer, including, but not limited to, polyolefins, such as polyethylene, polypropylene, and blends thereof. In one embodiment, the microporous film is made from a blend of a linear low-density polyethylene, a low density polyethylene, and a calcium carbonate filler. Additionally, other components, such as antioxidants and pigments, may be added to the blend.

Microporous films typically are made by incorporating filler particles into a polymer and stretching the resulting material to form a film having voids induced by the filler particles. Incorporating filler particles into a polymer introduces a range of variables for consideration. Such variables include the type of filler, the amount of filler, the filler particle size and size distribution, surface modifications of the filler particles, the mode or method of stretching the film, and the like. Each of these variables can affect the morphology and properties of the resulting microporous film. In the process of making the film, the components are blended, extruded, and embossed. The resultant film can then be stretched and heat cured Such methods are taught in United States Patent No. 4,777,073.

The absorbent nonwoven fabric may be made from various materials including rayon fibers; natural fibers, such as, but not limited to, cotton fibers and wood pulp fibers; synthetic fibers, such as, but not limited to, polyester fibers, polyamide fibers, and polyolefin fibers, and combinations thereof The fibers may be bicomponent fibers. The bicomponent fibers may be in a sheath-core configuration in which the sheath comprises one polymer and the core comprises a different polymer. Bicomponent fibers having other configurations, e.g., a side-by-side configuration may also be used.

The absorbent nonwoven fabric is typically a nonwoven made of solid fibers, however, the fibers, or a portion thereof, may be hollow fibers. Fibers having deniers ranging from about about 1 to about 15 may be advantageously used for the absorbent nonwoven fabric.

The absorbent nonwoven fabric may have multiple fibrous layers. The nonwoven may contain both absorbent fibers and synthetic non-absorbent fibers. Examples of synthetic non-absorbent fibers include, but are not limited to, polyethylene ("PE"), polypropylene ("PP"), polyethylene terephthalate ("PET"), bicomponent fibers, polyamides, and blends thereof Preferably, the nonwoven contains from about 70% to about 95% synthetic non-absorbent fibers by weight, based on the total weight of the nonwoven. As used herein, the term "absorbent fiber" means that the fiber has the ability to attract and retain liquid within the fiber matrix. Examples of suitable absorbent fibers include, but are not limited to, wood pulp fibers, cotton fibers, rayon fibers, and combinations thereof. Preferably, the nonwoven contains from about 5% to about 30% absorbent fibers, by weight, based on the total weight of the nonwoven. More preferably, the nonwoven contains from about 85% to about 90% by weight non-absorbent fibers and from about 10% to about 15% by weight absorbent fibers. The nonwoven can be produced with these fibers as a homogenous blend or, preferably as a layered blend within the nonwoven. In the latter case, one fibrous layer comprises all synthetic non-absorbent fibers. The other fibrous layer may contain all absorbent fibers or a blend of synthetic non-absorbent fibers and absorbent fibers. When a layered nonwoven fabric is employed, it is preferred that the layer made entirely from non-absorbent fibers be positioned next to the apertured film component of the inventive article. This layered blend arrangement does not generate high capillary pressures and does not draw too much moisture out of the wound.

The synthetic non-absorbent fibers can be of various deniers and types. The denier of the synthetic non-absorbent fibers may range from about 1.0 denier per filament ("dpf") to about 15 dpf, preferably from about 3 dpf to about 10 dpf. The basis weight of the nonwoven may range from about 30 grams per square meter ("gsm") to about 150 gsm depending on the product. If the inventive article is intended for use as a wound dressing, the basis weight of the nonwoven typically ranges from about 70 gsm to about 100 gsm. If the inventive article is intended for use as the wound contacting pad of an adhesive bandage, the basis weight of the nonwoven conveniently ranges from about 30 gsm to about 60 gsm. For more serious wounds that require higher absorption capacity, the basis weight of the nonwoven component of the inventive article may range from about 100 gsm to about 150 gsm.

A critical property in designing the nonwoven is the overall density of the nonwoven fabric. It is necessary that the nonwoven fabric comprising the absorbent article of the invention have a fabric density of from about 0.01 g/cc to about 0.05 g/cc. The density of the nonwoven fabric will vary based on the fiber types and deniers in addition to the thickness and basis weight.

Applicants are aware of two commercially available products which utilize in absorbent material. The first of these is marketed by 3M Company under the description NexCare* Triple Layer Gauze Pad. Despite the name given it, this product appears not to use Conventional gauze material. Rather, this product comprises a melt blown polyolefin nonwoven fabric covered on one side thereof by an apertured film and on the other side by a non-apertured barrier film. The melt blown nonwoven fabric has a density of about 0.08 g/cc. The second product of which applicants are aware is marketed by Johnson & Johnson Consumer Companies, Inc. under the designation Johnson & Johnson First Aid* Triple Layer Non-Stick Pads. This product comprises a carded cotton web wrapped in an apertured film. The carded cotton web has a density of 0.06 g/cc.

Although the above-mentioned densities of the absorbent materials of the two above-mentioned commercially available products are in the suitable ranges for drawing and absorption of fluid away from wounds, they are not optimum for providing a superior wound healing environment.

It has been found that when the nonwoven fabric comprising the absorbent article of the invention has a density ranging from about 0.01 g/cc to about 0.05 g/cc and comprises from about 70% to about 95% by weight of synthetic non-absorbent fibers and from about 30% to about 5% by weight of absorbent fibers, superior wound healing and case of removal are achieved while maintaining acceptable levels of absorbency. Such nonwoven fabrics are believed to exert reduced capillary pressure and allow the product to reduce the aggressiveness of liquid absorption, thereby keeping the wound moist. Moist wounds generally are preferred for better healing. The low capillary pressure produced by such nonwoven fabrics allows only excess fluid from the wound to be absorbed. In addition, because the wounds ate kept moist, the absorbent article does not stick to skin surfaces, including wounds, and is easier to remove without re-injury.

The apertured film and the optional backing material of the absorbent article of the invention may be secured to the absorbent nonwoven fabric by means known in the art. For example, a suitable adhesive may be applied to the first major surface of the absorbent nonwoven fabric, and the backing may then be applied to the absorbent nonwoven fabric. Then the second major surface of the absorbent nonwoven fabric may be coated with the adhesive and the apertured film may be applied to the absorbent nonwoven fabric, Other means known in the art, e.g. ultrasonic bonding, heat sealing, and the like, may also be used.

When utilized, the adhesives may be made from any polymerization process including solution or dispersion processes. The adhesives may be hot melt adhesives, Examples of suitable adhesives include, but are not limited to those based on styrenic block copolymers and tackifying resins such as HL-1491 from HB-Fuller Co. (St. Paul MN), H-2543 from ATO-Findley (Wawatausa, WI), and 34-5534 from National Starch & Chemical (Bridgewater, NJ). Ethylene copolymers, including ethylene vinyl acetate copolymers, may be used. Suitable adhesives also include acrylic based, dextrin based, and urethane based adhesives. Adhesives based on natural and synthetic elastomers are also suitable. Other adhesives which can be used include amorphous polyolefins, including amorphous polypropylene such as HL-1308 from HB Fuller or REXTAC RT 2373 from Huntsman (Odcsssa, TX). The adhesive may, if desired, be modified with KRATON® Brand synthetic rubber polymers or with natural rubber optionally containing tackifiers, antioxidant, and processing aids.

The adhesive can be applied in the molten stage, sprayed, or slot die coated. The spray can be applied by control coating, control weaving, control fiberization, meltblowing, flexo coating, screen printing, or other discontinuous coating methods.

The present invention also provides a method for making the absorbent article of the invention. The method includes: placing an apertured film having protuberances on a surface with the protuberances facing up; applying an adhesive to a first major surface of an absorbent nonwoven fabric having a density of from about 0.01 g/cc to about 0.05 g/cc; placing the adhesive coated first major surface of the absorbent nonwoven fabric on the apertured film and allowing the adhesive to set.

If the absorbent article is to include the aforementioned top layer, an adhesive is applied to one major surface of said top layer and the adhesive coated major surface of the top layer is brought into contact with the second major surface of the absorbent nonwoven fabric and the adhesive is allowed to set.

The absorbent article of the invention may be made in any desired shape, including, but not limited to, round, oval, rectangular, square, and triangular. The size of the absorbent article of the invention is not critical and may be varied depending on the desired application.

Absorbent articles of the invention may be used as is, for example, as wound coverings. The absorbent articles of the invention may also be used as a component of various products including, but not limited to, wound contacting pads for adhesive bandages, sanitary protection pads, disposable diapers, and implements for carrying and/or dispensing anti-itch agents, acne treating agents, moisturizers, and the like. For adhesive bandages, the absorbent article of the invention may be square, rectangular, round, oval, or triangular in shape. The size of the adhesive bandage will depend on the shape of the adhesive bandage and the size of the wound meant to be covered by the adhesive bandage. Generally, a square adhesive bandage may range in size from 5 cm x 5 cm to 15 cm x 15 cm, preferably from 7.5 cm x 7.5 cm to 12.5 cm x 12.5 cm. The length of a rectangular adhesive bandage may range from 5 cm to 15 cm, preferably from 7.5 cm to 12.5 cm. The width of a rectangular adhesive bandage may range from 0.5 cm to 5 cm, preferably from 1 cm to 3 cm. A circular adhesive bandage may range in outer diameter from 5 cm to 20 cm, preferably from 7.5 cm to 17.5 cm, more preferably from 10 cm to 15 cm.

The thickness of the absorbent article of the invention will vary depending on the application, but generally may range from 0.25 mm to 5 mm, preferably 1 mm to 3 mm, more preferably 1 mm to 2 mm.

When the absorbent article of the invention is used as the wound contacting pad of an adhesive bandage, the nonwoven fabric component of the absorbent article is secured, for example with an adhesive, to the backing material of the bandage. This leaves the apertured film component of the absorbent article facing upwardly, i.e., away from the adhesive coated surface of the backing material, in the finished bandage. Thus, when the adhesive bandage is used, the apertured film of the absorbent article is oriented toward the user's skin and serves as a wound release layer, meaning that the layer will not stick to the wound to which the adhesive bandage is applied.

If the inventive 2-layered absorbent article is used as the wound contacting pad of an adhesive bandage comprising an apertured backing material, the open area of the apertured film of the 2-layered absorbent article and the open area of the apertured backing material may be the same or may be different. In order to reduce the contact area of the wound contacting pad of the adhesive bandage against the wound, the open area of the apertured film of the absorbent article ranges from about 1 percent to about 35 percent, preferably from about 4 percent to about 30 percent of the total area of the apertured film. The reduction in contact area against the wound reduces the wound release force. This results in a lower re-injury occurrence upon removal of the bandage. The apertured backing material of the bandage may have a smaller open area than the apertured film of the absorbent article. The use of an apertured backing material having a reduced open area tends to prevent undesirable escape of liquid from the absorbent nonwoven material.

As mentioned above, the absorbent article of the invention may be used as the wound contacting pad of an otherwise conventional adhesive bandage. In a specific embodiment of such an adhesive bandage, a 2.54cm x 7.6cm strip of a polyethylene foam serves as the backing material of the adhesive bandge. The strip of polyethylene foam is coated with a suitable pressure sensitive adhesive. A 2.54cm x 2.54cm piece of the inventive article is centered over and then secured to the adhesive coated foam strip. Protective release tabs are secured to the adhesive on each side of the wound contacting material. The completed adhesive bandage is then wrapped, e.g., in a paper wrapper and, if desired, sterilized, The density of the foam strip may range from 0.008 g/cm³ to 0.160 g/cm³. The foam strip may be perforated or apertured. The size of the perforations or apertures may range from 0.01 mm to 5 mm. The total open area of the perforated or apertured foam strip may range from 10 percent to 80 percent. The perforations or apertures may be made by, e.g., hot-pin perforation or ultrasonic perforation. The foam backing material may be made from plastic materials other than polyethylene. It will be apparent to the those skilled in the art that other backing materials, such as polymeric films, woven fabrics, gauze, paper nonwoven fabrics and the like may be used as backing materials in place of the foam strip.

### Brief Description of the Drawings

FIG. is an exploded crors-seelional view of one embodiment of an absorbent article in accordance with the present invention.

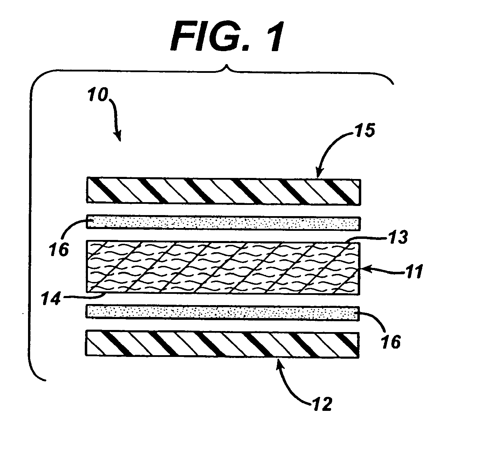

Referring now to the accompanying drawing, there is shown one embodiment of an absorbent article in accordance with the teachings of the present invention. Referring to FIG. 1, absorbent article 10 comprises an absorbent nonwoven fabric 11 and an apertured film 12, Absorbent nonwoven fabric 11 has a first major, or upper, surface 13 and a second major, or lower, surface 14. Apertured film 12 is secured, e.g., by adhesive 16, to lower surface 14 of the absorbent nonwoven fabric 11. A top layer 15 is optional. When present, top layer 15 is secured, e.g., by adhesive 16, to upper surface 13 of absorbent nonwoven fabric 11. Those skilled in the art will understand that top layer 15 and apertured film 12 can be secured to absorbent nonwoven fabric 11 by means other than adhesive, for example, by heat-sealing, ultrasonic bonding or the like. When adhesive is used, it is preferably applied in discontinuous fashion, for example, by spraying, screen printing, gravure printing or the like. Similarly, heat-sealing or ultrasonic bonding are preferably done in a discontinuous manner.

Absorbent nonwoven fabric 11 may comprise a single fibrous web of any desired thickness. Alternatively, absorbent nonwoven fabric 11 may comprise two or more such webs.

Top layer 15, if used, may be the same material as apertured film 12. Preferably, top layer 15 is a microporous film.

Absorbent article 10 shown in Fig. 1 may be used, with or without top layer 15, as a wound dressing or covering. When so used, the absorbent article can be held in place with, for example, strips of adhesive tape. In addition, absorbent article 10 can be used as the wound contacting pad of an otherwise conventional adhesive bandage as explained hereinabove.

The following Examples are intended to illustrate the absorbent article of this invention. The Examples should not be construed as limiting the scope of the invention.

### Example 1

A RETICULON* Brand apertured film, Code 6012, obtained from PGI Nonwovens was utilized to prepare a wound contacting pad for an adhesive bandage. This film had smooth upper and lower surfaces. The open arc of this film was about 28 percent.

The absorbent nonwoven fabric utilized in this Example 1 was a two-layer structure, Code 171-6-6, available from PGI Nonwovens. The top layer of this absorbent nonwoven fabric, which was positioned next to the above-mentioned apertured film, consisted entirely of 3.0 dpf polyethylene ("PE") sheath/polyethylene terephthalate ("PEI") core bicomponent fibers. The top layer of the Code 171-6-6 nonwoven had a basis weight of 50 grams per square meter. The second layer of the absorbent nonwoven fabric had a basis weight of 40 gsm and comprised a blend of 90% by weight 3.0 dpf bicomponent PE/PFT fibers and 10% by weight 1.5 dpf rayon fibers. The density of the Code 171-6-6 nonwoven fabric was 0.035 grams per cubic centimeter (g/cc).

RETICULON* Brand apertured film, Code 6012, was also utilized as a top layer 15 for the wound contacting pad of this Example 1.

### Example 2

TRBDEGAR apertured film, Code X-6944, was utilized to make the wound contacting pad of this Example 2. One major surface of this apertured film comprised protuberances which were oriented such that they faced into the absorbent nonwoven fabric described below. This apertured film had an open area of about 13%.

The absorbent nonwoven fabric utilized in this Example 2 was the same two-layer structure, Code 171-6-6, used in Example 1. The top layer of the absorbent nonwoven fabric was positioned next to the protuberances of the apertured film

A microporous polyethylene film, Code X617 W, from Tredegar Inc. was utilized as top layer 15 for the wound contacting pad.

### Example 3

This Example 3 utilized the same apertured film, i.e, TREDEGAR film, Code X-6944, as Example 2. The protuberances of this film faced into the absorbent nonwoven fabric, which was the same fabric, i.e., Code 171-6-6, which was used in above Example 2.

TREDEGAR film, Code X-6944, was utilized as top layer 15 for the wound contacting pad.

### Example 4

A poly(ethylene-methyl acrylate) ("EMA") film of the type specifically disclosed and illustrated in FIGS. 2 and 3 of United States Patent No. 6,566,577 and made by the mesh perforation technique disclosed in United States Patent No. 3,054,148 was utilized as the apertured film in this Example 4. The apertured film was produced by mesh-perforating an EMA film over a woven belt. The top surface of the mesh-perforated EMA film comprised land portions (identified by,numeral 6 in FIG. 2 of the '577 patent). The back surface of this EMA film was smooth as seen in FIG. 3 of the '577 patent. The EMA film was positioned such that its smooth surface came into contact with one of the major surfaces of the absorbent nonwoven fabric described below. The EMA film had an open area of 4.2%.

The absorbent nonwoven fabric utilized for this Example 4 was the same two layer structure, i.e., Code 171-6-6, used in Example 1.

A microporous polyethylene film, Code X617 W, from Tredegar Inc. was utilized as top layer 15 for the wound contacting pad of this Example 4.

### Example 5

TREDEGAR apertured film, Code X-6944, the same as the one used in Example 2, was utilized to make the wound-contacting pad of this Example 5. The apertured film was oriented so that its protuberances faced into the nonwoven fabric described below.

The absorbent nonwoven fabric utilized in this Example 5 was a two-layer structure, Code 171-6-5, obtained from PGI Nonwovens. The top layer of this absorbent nonwoven fabric consisted entirely of 3.0 dpf PE/PET bicomponent fibers. The top layer of the Code 171-6-5 fabric had a basis weight of 80 gsm. The second layer of the absorbent nonwoven fabric had a basis weight of 40 gsm and comprised a blend of 90% by weight of 3.0 dpf bicomponent PE/PET fibers and 10% by weight of 1.5 dpf rayon fibers. This two-layer nonwoven fabric had a density of 0.046 g/cc.

A microporous polyethylene film, Code X617 W, obtained from Tredegar was utilized as top layer 15 for the wound contacting pad.

### Example 6

TREDEGAR apertured film, Code X-6845, was utilized to make the wound-contacting pad for this Example 6. This film had an open are of about 13%. One major surface of this apertured film comprised protuberances which were oriented such that they faced into the absorbent nonwoven fabric. The absorbent nonwoven fabric utilized in this Example was a two-layer structure, Code 171-6-4, available from PGI Nonwovens. The top layer of this absorbent nonwoven fabric consisted entirely of 10.0 dpf PE/TET bicomponent fibers and had a basis weight of 25 gsm. The second layer of the absorbent nonwoven fabric had a basis weight of 25 gsm and contained a blend of 90% by weight of 3.0 dpf bicomponent PE/PET fibers and 10% by weight of 1.5 dpf rayon fiber. The density of the Code 171-6-6 nonwoven fabric was 0.025 g/cc.

A non-apertured polyolefin film was used as top layer 15.

### Process for Making Samples

The apertured film selected for each preceding Example was adhesively laminated to one major surface of the selected absorbent nonwoven fabric. For films with protuberances, the protuberances were aligned to face into the top layer of the absorbent nonwoven fabric. In each Example, the selected film and the selected nonwoven were laminated utilizing a Nordson ionizing spray system for hot melt adhesives. The hot melt adhesive used for these Examples was ATO-Findley 3210-02. In the same manner, the top layer 15 was then laminated to the other major surface of the nonwoven fabric. If top layer 15 comprised an apertured film with protuberances, the protuberances were faced into the absorbent nonwoven fabric.

### Density Measurements

A 2 inch X 2 inch sample of absorbent nonwoven fabric was cut and placed under an Ames thickness gauge (Model number 94-077). The foot of the thickness gauge was lowered onto the test specimen, said foot applying a pressure of 0.08 pounds per square inch ("psi"). The thickness of the sample was recorded. The sample was then placed on a balance and weighed. The density of the absorbent nonwoven fabric was determined by dividing the sample weight by the area and thickness of the sample.

### Capacity Measurement

A 3 inch x 4 inch sample was weighed and placed in a beaker containing 400 milliliters ("ml") of a 1% by weight saline solution. The sample was soaked in the saline for 10 minutes. The sample was removed and hung for 2 minutes to allow excess saline solution to drip off. The sample was placed on a horizontal surface and a piece of Whatman Code 1001-918 Filter Paper, available from VWR, Batavia, IL, was placed on the sample. A weight of 1.5 grams was placed on top of the filter paper for 30 seconds. After 30 seconds the weight was removed and the filter paper weighed. The filter paper was then placed again on the sample for 30 seconds and reweighed. The procedure was repeated until the successive differences between each weighing of the filter paper was less than 0.5 grams. Once this equilibrium was reached, the sample was weighed. The final capacity was calculated by subtracting the dry weight of the test sample from the wet weight of the equilibrated sample.

### Wound Release Test

For adhesive bandages, it is important that the layer of the adhesive bandage that contacts the wound does not stick to the wound. A wound release test is utilized to determine the quality of the adhesive bandage, in terms of not sticking to the wound. The absorbent articles prepared in the Examples were tested and compared to two commercially available products known for good wound release characteristics. The test was performed on female Yorkshire swine. On the day before the test, the animal was sedated and fur was removed from the animal's back and flank with electric clippers. A depilatory lotion was applied to the area. After 10 minutes, the lotion was removed with a metal spatula. The area was washed with mild soap and swabbed dry. The next day, the animal was sedated and a single row of twelve 2.5 cm x 2.5 cm superficial wounds was created on each flank, parallel to the spinal column using a Brown Dermatome set at a depth of 0.08 cm. The wounds were wiped with a gauze sponge and the bandages were applied perpendicular to the spinal column. A stretch bandage was applied over the test bandages to prevent movement or removal of the bandages.

Approximately 24 hours later, the animal was sedated. A Chatillon Digital Force Gauge Model DFGS2 with a chromalography clip attached to the bottom post of the unit by 5 cm of string was used to measure the bandage removal force. The protective stretch bandages were removed from the animal. The adhesive portions of the bandages under test were cut and removed from the animal, leaving only the wound contacting pad on the wound. The chromatography clip was attached to the top or bottom edge of each bandage and the force gauge was zeroed- The gauge was lifled perpendicular to the plane of the wound surface until the string was taught- The gauge was pulled back at a steady even rate until the bandage was removed from the wound surface. The force in grams) required to remove each test pad (Mean Removal Force) was recorded. The lower the force required to remove the pad, the better the wound release properties of the pad. Visual observations were also made on how moist the wound appeared after removing the wound contacting pad. The results of tests are shown in Tables 1 and 2.

**Table 1**

| **Sample** | **Removal Force (g)** | **Nonwoven Fabric Density (g/cc)** | **Capacity (g)** |
|---|---|---|---|
| 3M Triple Layer Gauze Pads | 96.3 | .08 | 1.5 |
| Johnson & Johnson First Aid Triple Layer Nonstick Pads | 353 | .06 | 4.0 |
| Example 1 | 282 | .035 | 4.0 |
| Example 2 | 112 | .035 | 4.0 |
| Example 3 | 98 | .035 | 4.0 |
| Example 4 | 80 | .035 | 4.0 |
| Example 5 | 111 | .046 | 8.0 |
| Example 6 | 108 | .025 | 1.0 |

Although not optimal, the sample of Example 1 shows improvement over the commercially available Johnson & Johnson Nonstick pad comparative sample. The improvement resulted from changing the density and the materials of construction of the nonwoven fabric. Examples 2, 3, and 4 show improved properties based on combining apertured films with the low density nonwoven fabric. Example 5 has a slightly higher density and weight of nonwoven fabric than Examples 2,3, and 4. Test results show very high capacity and low removal force. This type of sample is excellent for severe wound type products or sanitary protection products that require higher absorbent capacities. Example 6 is an example of an apertured film and a very low density nonwowen fabric. This type of sample is particularly useful for adhesive bandages. This sample also shows excellent wound release properties.

**Table 2**

| **Sample** | **Removal Force (g)** | **Capacity (g)** | **Moist Wound Appearance (%)** | **Wounds Showing moderate Re-Injury (%)** |
|---|---|---|---|---|
| Example 1 | 282 | 4.0 | 58 | 8 |
| Example 4 | 80 | 4.0 | 100 | 0 |

Table 2 shows that as improvements are made in wound release force with Examples 1 and 4, the appearance of moist wounds is higher and the re-injury upon removal of the pad is reduced.

## Claims

1. An absorbent article, comprising:
an absorbent nonwoven fabric having a density from 0.01 g/cc to 0.05 g/cc, said nonwoven fabric comprising a first fibrous layer consisting of synthetic non-absorbent fibers and a second fibrous layer comprising absorbent fibers; and
an apertured film secured to said nonwoven fabric, wherein said first fibrous layer is positioned next to said apertured film.

2. The article of claim 1 wherein said non-absorbent fibers are bicomponent fibers.

3. The article of claim 1 wherein said nonwoven fabric comprises a blend of from 70% to 95% by weight of said synthetic non-absorbent fibers and from 5% to 30% by weight of said absorbent fibers and has a basis weight ranging from 30 gsm to 150 gsm.

4. The article of claim 1 wherein said apertured film comprises a polymeric material selected from the group consisting of ethylene methyl acrylate, polyethylene, metallocene catalyzed polyethylene, polypropylene, and copolymers thereof, and ethylene vinyl acetate copolymers.

5. The article of claim 1 wherein said article is a wound contacting pad.

6. The article of claim 5 wherein the open area of said apertured film ranges from 5 percent to 30 percent of the total area of said apertured film.

7. The article of claim 1 further comprising a top layer secured to said second fibrous layer.

8. The article of claim 7 wherein said top layer comprises a microporous film.

9. The article of claim 8 wherein said article is selected from the group consisting of a disposable diaper, a sanitary protection pad and an adhesive bandage.

## Patentansprüche

1. Absorbierender Gegenstand, der aufweist:
einen absorbierenden Vliesstoff mit einer Dichte von 0.01 g/cm³ bis 0.05 g/cm³, wobei der Vliesstoff eine erste faserige Schicht, die aus synthetischen, nicht absorbierenden Fasern besteht, und eine zweite faserige Schicht, die absorbierende Fasern aufweist, aufweist; und
einen gelochten Film, der an dem Vliesstoff gesichert ist, wobei die erste faserige Schicht nahe dem gelochten Film angeordnet ist.

2. Gegenstand nach Anspruch 1, bei dem die nicht absorbierenden Fasern zweikomponentige Fasern sind.

3. Gegenstand nach Anspruch 1, bei dem das Vlies eine Mischung aus 70 bis 95 Gew.% der synthetischen, nicht absorbierenden Fasern und 5 bis 30 Gew.% der absorbierenden Fasern aufweist und ein Riesgewicht hat, das im Bereich von 30 g/m² bis 150 g/m² liegt.

4. Gegenstand nach Anspruch 1, bei dem der gelochte Film ein polymeres Metall aufweist, das aus der Gruppe bestehend aus Ethylenmethylacrylat, Polyethylen, mit Metallocen katalysiertem Polyethylen, Polypropylen und Copolymeren aus diesen und Ethylvinylacetat-Copolymeren ausgewählt ist.

5. Gegenstand nach Anspruch 1, wobei der Gegenstand ein eine Wunde berührendes Kissen ist.

6. Gegenstand nach Anspruch 5, bei dem die offene Fläche des gelochten Films im Bereich von 5 Prozent bis 30 Prozent der Gesamtfläche des gelochten Films liegt.

7. Gegenstand nach Anspruch 1, der weiter eine obere Schicht aufweist, die an der zweiten faserigen Schicht gesichert ist.

8. Gegenstand nach Anspruch 7, bei dem die obere Schicht einen mikroporösen Film aufweist.

9. Gegenstand nach Anspruch 8, bei dem der Gegenstand aus der Gruppe bestehend aus einer Wegwerfwindel, einem hygienischen Schutzkissen und einer Klebbandage ausgewählt ist.

## Revendications

1. Article absorbant comprenant :
un tissu non tissé absorbant ayant une densité de l'ordre de 0,01 g/cc à 0,05 g/cc, ledit tissu non tissé comprenant une première couche fibreuse constituée de fibres non absorbantes synthétiques et une seconde couche fibreuse comprenant des fibres absorbantes ; et
un film perforé fixé audit tissu non tissé, ladite première couche fibreuse étant placée à proximité dudit film perforé.

2. Article selon la revendication 1, dans lequel lesdites fibres non absorbantes sont des fibres à deux composants.

3. Article selon la revendication 1, dans lequel ledit tissu non tissé comprend un mélange de 70 % à 95 % en poids desdites fibres non absorbantes synthétiques, et de 5 % à 30 % en poids desdites fibres absorbantes et a un poids de base allant de 30 gsm à 150 gsm.

4. Article selon la revendication 1, dans lequel ledit film perforé comprend un matériau polymère choisi dans le groupe comprenant un éthylène méthyle acrylate, un polyéthylène, un polyéthylène obtenu par catalyse métallocène, un polypropylène et des copolymères de ceux-ci, et des copolymères d'éthylène acétate de vinyle.

5. Article selon la revendication 1, dans lequel ledit article est une compresse de contact avec une plaie.

6. Article selon la revendication 5, dans lequel la surface ouverte dudit film perforé va de 5 pour cent à 30 pour cent de la surface totale dudit film perforé.

7. Article selon la revendication 1, comprenant en outre une couche supérieure fixée sur ladite seconde couche fibreuse.

8. Article selon la revendication 7, dans lequel ladite couche supérieure comprend un film microporeux.

9. Article selon la revendication 8, dans lequel ledit article est choisi dans le groupe comprenant une couche jetable, une serviette hygiénique et un pansement adhésif.
